(19) Europäisches Patentamt
European Patent Office
Office européen des brevets

(11) Numéro de publication : **0 465 295 A1**

(12) # DEMANDE DE BREVET EUROPEEN

(21) Numéro de dépôt : **91401697.7**

(51) Int. Cl.[5] : **C07K 5/06**, A61K 49/00

(22) Date de dépôt : **24.06.91**

(30) Priorité : **26.06.90 FR 9008009**

(43) Date de publication de la demande :
**08.01.92 Bulletin 92/02**

(84) Etats contractants désignés :
**AT BE CH DE DK ES FR GB GR IT LI LU NL SE**

(71) Demandeur : **MEDGENIX GROUP, S.A.**
**1, place d'Italie**
**B-4020 Liège (BE)**

(72) Inventeur : **Chiu, Kwok Wai**
**152 High Street**
**Gloucester GL1 4TF (GB)**
Inventeur : **Hussain, Wasif**
**Chaussée d'Alsemberg 1031**
**B-1180 Uccle (BE)**
Inventeur : **Thornback, John Richard**
**11 rue des Papeteries**
**B-1325 Chaumont-gistoux (BE)**

(74) Mandataire : **Warcoin, Jacques**
**Cabinet Régimbeau 26, avenue Kléber**
**F-75116 Paris (FR)**

(54) **Agent de contraste pour l'imagerie par RMN et agents chélateurs comportant des structures peptidiques.**

(57) La présente invention a pour objet un agent de contraste pour l'imagerie par RMN constitué d'un complexe entre un agent chélatant et des cations de métal paramagnétique, caractérisé en ce que ledit complexe répond à la formule I :

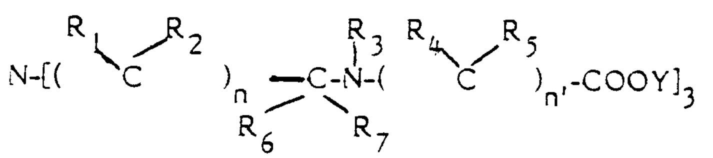

I

ou un sel physiologiquement acceptable de ce complexe, formule dans laquelle

— n, n' sont des nombres entiers identiques ou différents de 1 à 4

— les n et n' ($R_1$, $R_2$) et ($R_4$, $R_5$) respectivement identiques ou différents, $R_3$ et ($R_6$, $R_7$) sont choisis parmi H, un halogène, les groupes alkyl, alcoxy, aryl, alkényl, cycloalkyl éventuellement substitués par des groupes halogènes alcoxy, alkyl, nitril, nitro ou amino,

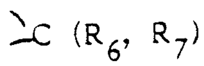

pouvant en outre représenter

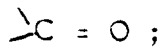

— les trois substituants Y sont identiques ou différents et représentent H ou un équivalent ionique de cation de métal paramagnétique divalent ou trivalent ou d'un cation physiologiquement acceptable provenant d'une base minérale ou organique,

avec la condition que deux au moins des substituants Y représentent des équivalents ioniques de cations de métaux paramagnétiques.

EP 0 465 295 A1

La présente invention concerne des agents de contraste pour l'imagerie par RMN constitués d'un complexe entre un agent chélatant et des cations de métal paramagnétique. La présente invention concerne également des agents chélatants de structure peptidique pouvant former des complexes métalliques, ainsi que des procédés de préparation desdits agents de contraste ou agents chélatants.

Il est connu depuis longtemps que l'on peut utiliser dans le domaine de l'imagerie par résonnance magnétique (IRM) des ions paramagnétiques pour favoriser la relaxation du spin de certains noyaux atomiques (Bloch F., Hancen W.M. et Packard N., Phys. Rev. 1946, 70, 474-485). Pour que le phénomène du RMN soit observable, il faut que l'échantillon contienne un nombre important de noyaux possédant un moment magnétique ; c'est le cas de l'atome d'hydrogène $^{1}H$ (protons de l'eau), du phosphore $^{31}P$ et du carbone $^{13}C$. En IRM, les spins des noyaux atomiques du tissu examiné sont orientés dans une certaine direction au moyen d'un champ magnétique principal. Cette orientation est ensuite perturbée par l'application d'un champ électromagnétique de fréquence déterminée (processus d'excitation). Quand le second champ est interrompu, l'énergie absorbée par la matière lors du processus d'excitation est restituée par les noyaux dont le moment magnétique va à nouveau s'aligner avec la direction du champ magnétique principal. L'énergie émise, dite énergie de précession décroît selon un phénomène de relaxation. La relaxation se décompose en deux processus :

– une relaxation spin-spin caractérisée par une constante de temps $T_2$,
– une relaxation spin-réseau caractérisée par une constante de temps $T_1$.

L'intensité du signal émis dépend, notamment de T1 et T2. Le contraste entre deux tissus différents dépend donc de la différence entre T1 et T2 de ces deux tissus.

En IRM, les séquences d'acquisition des signaux pondérés en $T_1$ ou en $T_2$ permettent d'obtenir des informations sur la morphologie et la physiologie des organes étudiés mais aussi sur leurs compositions.

Pour que le phénomène de RMN soit observable, il faut que l'échantillon contienne un nombre important de noyaux possédant un moment magnétique, c'est le cas en particulier de l'atome d'hydrogène $^{1}H$, du phosphore $^{31}P$ et du carbone $^{13}C$.

Cependant, l'IRM se heurte à un problème de sensibilité et de contraste. Les agents paramagnétiques permettent d'augmenter ce contraste : en effet, ils modifient les T1 et T2 des noyaux des tissus où ils s'accumulent et donc l'intensité du signal émis par ces tissus. Outre l'information anatomique, les agents de contraste paramagnétiques donnent également une information fonctionnelle des tissus où ils s'accumulent. Ils sont donc utiles pour le diagnostic médical.

Les complexes de chélates et de métaux paramagnétiques sont les agents de contraste les plus fréquemment employés dans l'imagerie par RMN dans le domaine du diagnostic médical.

La Société Shering AG a décrit dans le brevet US 4 647 447 la préparation et l'emploi en imagerie d'agents de contraste RMN, et notamment le Gd $(DTPA)^{2-}$ (Gadolinium diéthylène-triamine pentaacétic acid). Ce complexe présente l'avantage d'être très stable, peu toxique et bien toléré. En revanche, il ne présente d'affinité pour aucun organe cible particulier de l'organisme. Il est excrété par voie urinaire et peut être utilisé, notamment, pour les examens rénaux.

Parmi les agents de contraste classiques, on peut également citer les colloïdes super-paramagnétiques de type dextran-oxyde de fer. Ces colloïdes sont rapidement captés par le système réticulo-endothélial permettant ainsi le diagnostic au niveau hépatique et splénique.

A ce jour, aucun chélate paramagnétique soluble et injectable n'a été décrit comme agent de contraste RMN pour une application en imagerie du foie.

Le but de la présente invention est de proposer un agent de contraste du type chélate paramagnétique soluble en solution aqueuse pouvant être administré par voie parentérale, par exemple par injection intraveineuse, de façon à obtenir un effet de contraste en imagerie par RMN d'un organe spécifique, notamment du foie. Plus précisément, le but de la présente invention est de proposer un agent de contraste qui, après adminstration à faible dose, conduit à un accroissement spécifique de la relaxation du proton de l'eau dans un organe particulier sans interférer avec d'autres organes et sans causer d'effets secondaires.

Un autre but est que l'agent de contraste présente à la fois une haute stabilité, une faible toxicité, c'est-à-dire soit physiologiquement tolérable d'une part, et que d'autre il présente les propriétés de relaxivité et d'osmolarité satisfaisantes pour une bonne imagerie par contraste.

Pour ce faire, la présente invention fournit un agent de contraste pour l'imagerie par RMN constitué d'un complexe entre un agent chélatant et des cations de métaux paramagnétiques, caractérisé en ce que ledit complexe répond à la formule I :

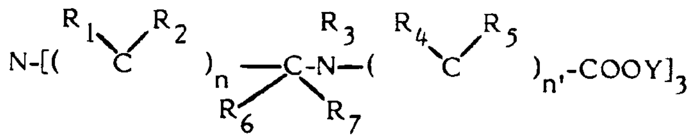

I

ou un sel physiologiquement acceptable de ce complexe, formule dans laquelle

– n, n' représentent des nombres entiers identiques ou différents de 1 à 4

– les n et n' ($R_1$, $R_2$) et ($R_4$, $R_5$) respectivement identiques ou différents entre eux, $R_3$, $R_5$ et $R_7$ sont choisis parmi H, un halogène, les groupes alkyl, alcoxy, aryl, alkényl, cycloalkyl, arylalkyl éventuellement substitués par des groupes halogènes alcoxy, alkyl, nitril, nitro ou amino ;

- 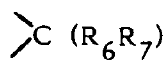 pouvant en outre représenter 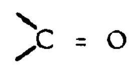

– les trois substituants Y sont identiques ou différents et représentent H ou un équivalent ionique de cation de métal paramagnétique divalent ou trivalent ou d'un cation physiologiquement acceptable provenant d'une base minérale ou organique,

avec la condition que deux au moins des substituants Y représentent des équivalents ioniques de cations de métaux paramagnétiques.

Ces complexes peuvent être neutres ou ioniques en fonction des charges respectives de l'agent chélatant et du cation du métal complexé.

Lorsque les atomes d'hydrogène acides des fonctions acides de l'agent chélatant ne sont pas tous remplacés par un ion paramagnétique, il est bon, pour augmenter la solubilité du complexe, de remplacer les atomes d'hydrogène restant par des cations inoffensifs du point de vue physiologique provenant de bases minérale et/ou organique, notamment d'amino acide.

La présentation du complexe sous forme salifiée est en général requise pour accroître la solubilité du complexe en phase aqueuse.

Selon la présente invention, les groupes alkyl, alcoxy, alkényl, sont des radicaux en $C_1$ à $C_7$ de préférence en $C_1$ à $C_4$. On citera en particulier les agents pour lesquels $R_1$ à $R_5$ représentent H, un halogène et un alkyl en $C_1$-$C_4$ éventuellement substitué par un ou des halogènes.

Les agents de contraste de l'invention sont particulièrement efficaces lorsque le métal paramagnétique est choisi parmi les ions divalents ou trivalents de métaux de transition ou de lanthanides paramagnétiques.

On peut citer plus particulièrement les ions suivants : $Co^{2+}$, $Mn^{2+}$, $Cu^{2+}$, $Cr^{3+}$, $Fe^{2+}$, $Fe^{3+}$, $Eu^{2+}$, $Gd^{3+}$, $Dy^{3+}$, $Ho^{3+}$.

On cite plus particulièrement les agents de contraste de formule I pour lesquels

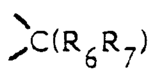

représente

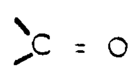

Avantageusement Y représente pour les 3 substituents un équivalent ionique de cation trivalent. C'est-à-dire que les atomes d'hydrogène acides de l'agent chélatant sont remplacés par un équivalent ionique trivalent paramagnétique.

C'est pourquoi, dans un mode de réalisation particulier des agents de contraste selon l'invention, ceux-ci ont pour formule II

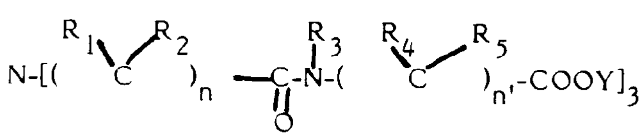

II

ou un sel d'acide physiologiquement acceptable de formule III

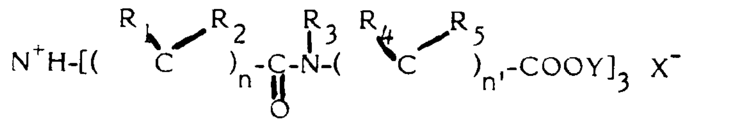

III

dans laquelle $X^-$ est l'anion provenant d'un acide de formule HX, formules dans lesquelles

– Y représente un équivalent ionique de métal paramagnétique trivalent,

– n, n', $R_1$, $R_2$, $R_3$, $R_4$, $R_5$ ont les significations données précédemment.

On utilisera de préférence les sels obtenus avec les anions $X^-$ qui représentent $Cl^-$, $Br^-$, $F^-$, $I^-$, $C_nH_{2n+1}$ $COO^-$, $C_6H_5COO^-$, $C_nF_{2n+1}$ $COO^-$, sont particulièrement solubles en phase aqueuse.

Parmi les agents de contraste selon l'invention, il convient de citer particulièrement les agents dans lesquels $R_3$ = H, ainsi que ceux pour lesquels $R_1$ , $R_2$, $R_4$ et $R_5$ = H, ainsi que ceux pour lesquels n = n' = 1.

De même, on citera plus particulièrement les sels obtenus avec $X^-$ = $CF_3COO^-$.

Enfin, sont particulièrement efficaces les agents de contraste utilisant le métal paramagnétique $Gd^{3+}$.

A titre d'exemple, on cite l'agent de contraste du foie dans lequel $R_1$ à $R_6$ = H, n = n' = 1 et $X^-$ = $CF_3COO^-$.

Les agents chélatants à structure peptidique utilisés dans les agents de contraste de formule II sont nouveaux, et par conséquent, constituent un autre aspect de la présente invention, laquelle a en effet également pour objet des composés répondant à la formule IV

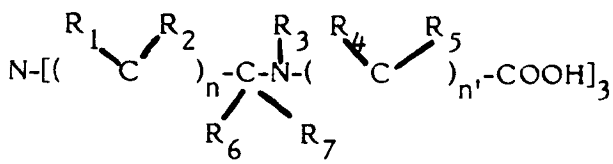

IV

ou un sel physiologiquement acceptable de celui-ci, formule dans laquelle n, n', $R_1$ , $R_2$, $R_3$, $R_{,4}$, $R_5$, $R_6$, $R_7$ ont les significations données précédemment. On cite en particulier les agents chélatants pour lesquels

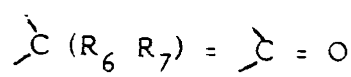

Dans un mode de réalisation particulier, les agents chélatants selon l'invention peuvent être solubilisés sous forme de sel de formule V

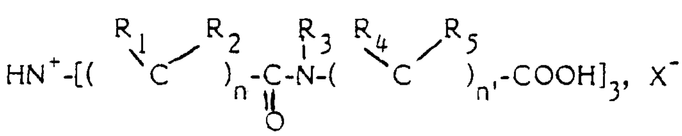

V

ou $X^-$ provient d'un acide HX tel que défini précédemment.

Ces agents chélateurs de formule IV ou V peuvent servir à former des complexes avec un ou des cations métallique(s) paramagnétiques ou non, radioactifs ou non, complexes pouvant avoir d'autres usages que l'application à titre d'agent de contraste pour RMN.

Selon un autre aspect, la présente invention a pour objet un procédé de préparation d'un agent de contraste pour l'imagerie par RMN suivant l'invention, caractérisé en ce que l'on mélange en solution aqueuse un agent chélateur de formule II dans laquelle Y = H ou un sel physiologiquement acceptable tel que défini précédemment avec un composé dudit métal paramagnétique au moins modérément soluble dans l'eau, par exemple un sel de métal paramagnétique soluble dans l'eau dont l'ion associé est physiologiquement acceptable, ou un carbonate ou un oxyde, au moins faiblement soluble, éventuellement en suspension.

Selon un autre aspect de la présente invention, celle-ci a pour objet un procédé de préparation d'un agent chélatant selon l'invention, caractérisé en ce que l'on fait réagir un composé de formule

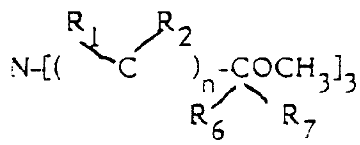

avec un composé de formule

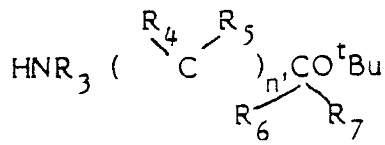

pour obtenir le composé de formule

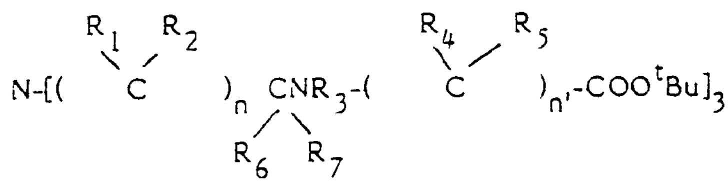

puis l'on déprotège les fonctions acides éventuellement en faisant réagir le composé avec un acide pour obtenir un sel d'ammonium.

D'une manière générale, les complexes, agents de contraste et agents chélateurs selon l'invention, sont préparés par des procédés qui mettent en oeuvre des réactions courantes.

De préférence, les agents de contraste selon l'invention sont en solution dans un solvant servant de véhicule physiologiquement acceptable, par exemple de l'eau pour injection.

Etant donné qu'il est préférable que l'agent de contraste selon l'invention se trouve au pH physiologique, la solution peut également contenir un tampon.

L'administration des agents de contraste selon l'invention s'effectue de préférence par injection intraveineuse d'une solution contenant le chélate paramagnétique en concentration qui suffit à conférer l'effet de contraste par imagerie RMN souhaité. A cet égard, des solutions contenant le métal paramagnétique en concentration de 0,2 à 200 mM sont appropriées.

En alternative, des agents de contraste selon l'invention peuvent être présentés sous des formes convenant à l'administration par la voie orale, par exemples des solutions, des comprimés ou des gélules.

Les agents de contrastes peuvent commodément être administrés en quantité qui fluctuent de $10^{-4}$ à $10^{-1}$ mmoles de métal paramagnétique par kilogramme de poids corporel.

Les expériences réalisées ont montré que les chélates selon l'invention étaient des agents de relaxation efficaces qui possédaient une spécificité essentiellement pour le foie, mais aussi pour les reins comme il apparaîtra dans les exemples qui suivent.

La qualité des images obtenues par RMN avec les chélates selon l'invention est bien meilleure que le standard général des images obtenues par RMN publiées dans la littérature.

Les chélates et agents de contraste selon l'invention seront davantage illustrés dans les exemples non limitatifs qui suivent.

La figure 1 représente l'image d'un organe de rat par IRM avec l'agent de contraste Gd (MNM-50) avant (colonne gauche) et après (colonne droite) administation dudit composé à une dose de 0,1 mmol/kg. L'image après administration du composé Gd (MNM-50) (colonne de droite) a été prise 10 minutes après injection. Les meilleurs signaux sont obtenus dans le foie et les reins.

**EXEMPLE 1 : SCHEMA GENERAL DES SYNTHESES**

La préparation des agents de contraste et des agents chélateurs selon l'invention peut se faire par exemple selon le schéma ci-après, lorsque

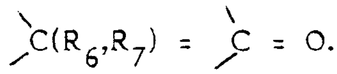

1) Préparation de :

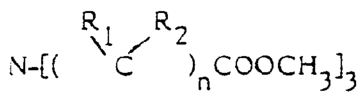

(1)

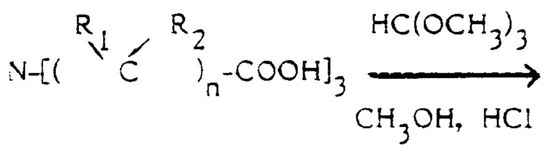


(1)

2) Préparation de :

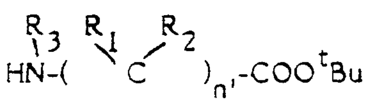

(2)

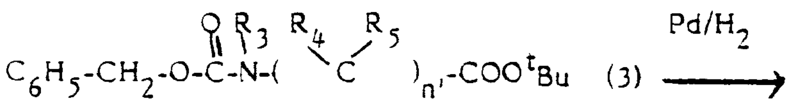


(3) (2)

3) Préparation de (3)

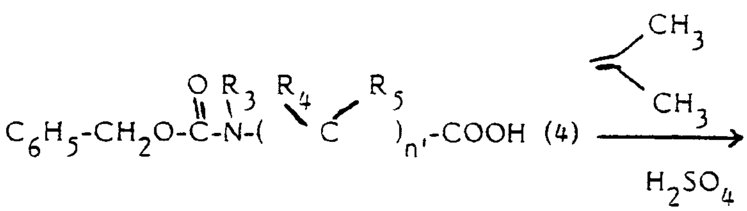


(4) (3)

4) Préparation de (4)

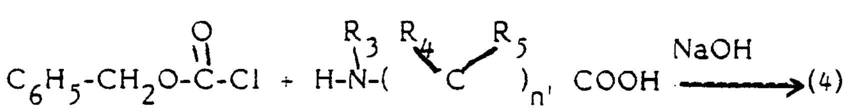


(4)

5) Préparation de I-1

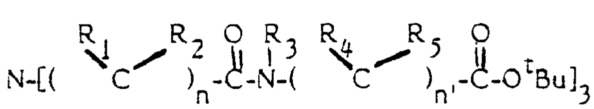

(I-1)

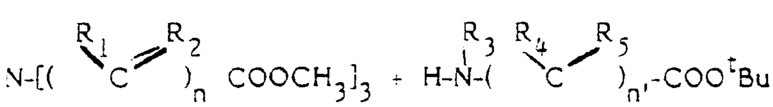

130°C 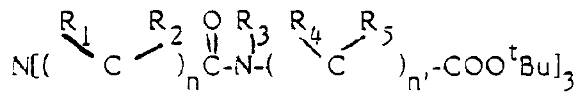 (I-1)

6) Préparation de II-1

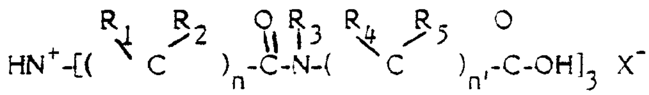 (II-1)

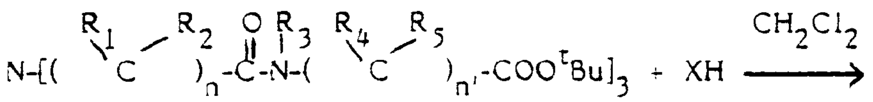 (II-1)

7) Préparation de III-1

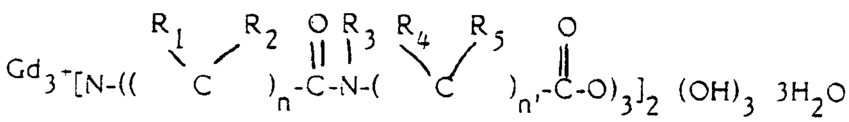 (III-1)

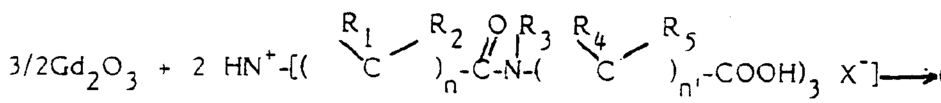 (III-1)

8) Préparation de IV-1

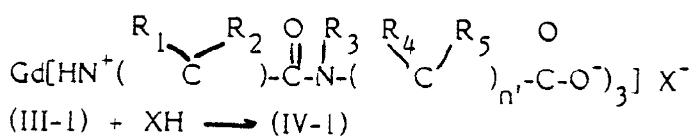 (IV-1)

(III-1) + XH → (IV-1)

**EXEMPLE 2 : PREPARATION DE L'INTERMEDIAIRE (1) DE FORMULE $N(CH_2CONHCH_2COO^tBu)_3$**

1) Préparation de $N(CH_2COOCH_3)_3$ (1)

$$N(CH_2COOH)_3 \xrightarrow[CH_3OH,\ HCl]{HC(OCH_3)_3} N(CH_2COOCH_3)_3 \quad (1)$$

A un mélange sous agitation d'acide nitrilotriacétique (38,2 g ; 0,2 moles) et d'orthoformate de triméthyle (84,8 g et 0,8 moles) dans du méthanol (160 ml), on ajoute de l'acide chlorhydrique gazeux (préparer à partir de 30 ml de HCl 12 M et 30 ml de $H_2SO_4$ 36 N) à température ambiante. Quand le mélange devient homogène, la réaction est maintenue à 65°C pendant 12 heures puis à température ambiante pendant 3 jours. La solution est portée à pH 10 par addition d'une solution d'hydroxyde de sodium dans du méthanol. Après concentration sous vide, le résidu est purifié par chromatographie sur colonne de silica gel avec comme éluant de l'acétate d'éthyle. Le triméthylnitriloacétate est obtenu sous forme de liquide incolore (40 g ; rendement 90 %). Données spectroscopiques, IR : 1735 $cm^{-1}$ ; $^1H$ NMR (360 MHz, $CDCl_3$) ; 3.72 (3H, s), 3,66 (2H, s) ppm.

2) Préparation de N-benzyloxycarbonylglycine

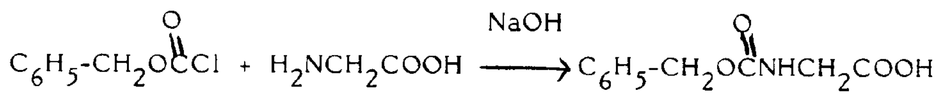


Dans une solution de glycine (15 g ; 0,2 moles) dans de l'hydroxyde de sodium aqueux (100 ml de solution de 2 N) bien agité et refroidi dans de la glace, sont ajoutés du chlorocarbonate de benzyle (37,4 g ; 0,22 moles) et de l'hydroxyde de sodium 2 N (110 ml) sont ajoutés en 5 portions à une vitesse telle que la température du mélange soit maintenue en dessous de 10°C. Ensuite, le mélange est agité à température ambiante pendant 18 heures.

Après extraction du mélange avec de l'éther (4 fois avec 100 ml à chaque fois), l'éther est éliminé de la phase aqueuse par barbotage d'azote. La solution est ensuite acidifiée à pH 3 avec de l'acide chlorhydrique (44 ml de HCl 5 M) après quoi une huile se sépare et se solidifie lentement. Le solide est filtré, lavé avec de l'eau et séché sous vide sur du pentoxyde de phosphore, donnant de la N-benzyloxycarbonylglycine incolore 38 g ; rendement 91 %).
Données spéctroscopiques, RMN $^1H$ (360 MHz, $CDCl_3$) : 7,35 (5H), 5,25 (1H, br), 5,15 (2H, s), 4,05 (2H) ppm.

3) Préparation d'ester de terbutyl et N-benzyloxycarbonylglycine

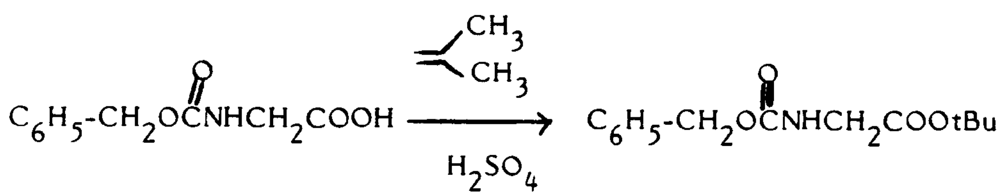


A une solution de N-benzyloxycarbonylglycine (24 g ; 0,12 moles) dans du dichlorométhane (240 ml), on ajoute de l'acide sulfurique concentré (1,5 ml). L'isobutylène obtenu à partir du traitement de tert-butanol (110 ml) et par de l'acide phosphorique à 85 % (40 ml) à 110°C suivi d'un piège à -78°C, est amenée dans le mélange réactionnel sous agitation. Le récipient fermé est agité pendant 3 jours à température ambiante.

La solution est lavée avec une solution aqueuse de carbonate de sodium (200 ml contenant 15 g), avec de l'eau (3 fois 10 ml chaque fois) et séchée sur sulfate de magnésium anhydre. Après filtration, la solution est concentrée sous vide pour donner l'ester de tert-butyl et N-benzyloxycarbonylglycine sous forme de liquide incolore (27,5 ; rendement 91 %).
Données spectroscopiques, RMN $^1H$ (360 MHz, $CDCl_3$) : 7,35 (5H), 5,20 (1H, br), 5,12 (2H, s), 3,87 (2H, d), 1,44 (9H, s) ppm.

4) Préparation d'ester de tert-butyl et de glycine

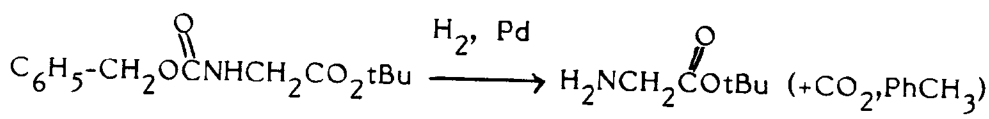


Une suspension bien agitée de tert-butylester (27,5 g ; 0,104 moles) et de Pd-C 10 % (2 g) dans du méthanol anhydre ( 150 ml) est traitée avec de l'hydrogène pendant 12 heures. Après élimination du catalyseur par filtration, le filtrat est concentré sous vide. Le résidu est repris à l'éther (150 ml), lavé avec du carbonate de sodium aqueux 10 % (2 fois 50 ml chaque fois) et séché sur sulfate de magnésium anhydre. Après filtration et concentration sous vide, le tert-butyl ester de glycine est obtenu sous forme de liquide incolore (9,1 g ; 67 % de rendement).

Données spectroscopiques, RMN $^1H$ (360 MHz, $CDCl_3$) : 3,30 (2H, s), 1,45 (9H, s) ppm.

5) Préparation de $N(CH_2CONHCH_2COO^tBu)_3$

$$H_2NCH_2COOtBu + N(CH_2COOCH_3)_3 \longrightarrow N(CH_2\overset{O}{\overset{\|}{C}}NHCH_2\overset{O}{\overset{\|}{C}}OtBu)_3$$

Un mélange sous agitation de tert-butyl ester de glycine (1,1 g ; 9 mmoles) et de triméthyl ester d'acide nitrilotriacétique (0,58 g ; 2,5 mmoles) est chauffé à 130°C pendant 7 heures. Le solide résultant est purifié par chromatographie sur colonne de silica gel en utilisant du méthanol 5 % dans du dichlorométhane comme éluant pour donner le triester recherché sous forme de solide jaune clair (0,82 g ; 63 %). Une purification ultérieure est effectuée par recristallisation à partir de chlorure de méthylène-hexane (m.p. 145 °C).

Données spectroscopiques, IR : 1740, 1680, 1645, 1530, 1155 $cm^{-1}$ ; RMN $^1H$ (360 MHz, $CDCl_3$) : 7,75 (3H, t), 3,96 (6H, s), 3,38 (6H, s), 1,45 (27H, s) ppm ; MS : m/z à 530 ($M^+$, 15), 457 (15), 399 (15), 287 (25), 260 (100), 246 (15), 145 (45), 57 (45).

**EXEMPLE 3 : PREPARATION DE L'INTERMEDIAIRE (II- 1)**

$[HN^+(CH_2CONHCH_2COOH)_3].CF_3COO^-$ (composé MNM-50) (II-1)

A une solution de $N(CH_2CONHCH_2COOBu^t)_3$, (I, 0,5 g, 0,93 mmoles) dans $CH_2Cl_2$ (24 ml) on ajoute du $CF_3COOH$ (24 ml). Le mélange réactionnel est agité à 20°C pendant 18 heures. Les matières volatiles sont éliminées par évaporation sous vide, puis du diéthyl éther est ajouté au résidu pour précipiter un solide blanc. Le solide blanc est recueilli par filtration, lavé à l'éther (5 fois 5 ml) et séché sous vide. Rendement = 0,38 g, 86 %.

Données spectroscopiques :

| | |
|---|---|
| RMN $^1H$ ($d^6DMSO$) | =3,58 singulet (2H)N-$C\underline{H}_2$ |
| | 3,85 doublet (2H)NH-$C\underline{H}_2$, ($^3J_{H-H}$ = 5,8 Hz) |
| | 8,58 singulet large (1H), N$\underline{H}$. |
| RMN $^{19}F$ ($^1H$) (DMSO) | =-74,5 ppm singulet. |

Microanalyses :

| | Trouvé | Théorique |
|---|---|---|
| C ; | 35,43 | 35,30 |
| H ; | 4,32 | 4,02 |
| N ; | 11,08 | 11,76 |

**EXEMPLE 4 : PREPARATION DE L'INTERMEDIAIRE (III-1)**

$Gd_3[N(CH_2CONHCH_2COO)^-_3]_2(OH)_3 3H_2O$ (III-1)

A une suspension de $Gd_2O_3$ (0,256 g, 0,7 mmoles) dans l'eau (150 ml) on ajoute le composé MNM-50 (II, 0,57 g, 1,57 mmoles).

Le mélange réactionnel est tout d'abord agité à 80°C pendant 1 heure puis la température est montée à 100°C et l'agitation est poursuivie pendant 4 heures. Après refroidissement, un solide blanc est filtré et lavé avec de l'eau, de l'éthanol et du pentane puis séché sous vide. Rendement : 0,5 g, 58 %.

Microanalyses :

| | Trouvé | Théorique |
|---|---|---|
| C : | 23,7 | 22,3 |
| H : | 3,8 | 3,01 |
| N : | 8,9 | 8,7 |

**EXEMPLE 5 : PREPARATION DU SEL COMPLEXE**

$Gd^{3+}[HN^{+}-(CH_2CONHCH_2COO^{-})_3]CF_3COO^{-}.1/2CF_3COOH.2H_2O$ (IV-1)

(Gd MNM-50)

Du $Gd_3[N(CH_2CONHCH_2COO^{-})_3]_2(OH)_33H_2O$ (III, 0,4 g, 0,72 mmoles) est dissout dans du $CF_3COOH$ (0,1 M, 100 ml) à 20°C ; tous les produits insolubles sont éliminés par filtration puis le filtrat est agité à 70°c pendant 1 heure, puis à 20°C pendant 18 heures. Les matériaux volatiles sont éliminés par évaporation sous vide. Le résidu huileux est lavé avec du diéthyl éther pour donner un solide blanc qui est isolé par filtration, lavé avec du diéthyl éther (10 fois 4 ml) et séché sous vide. Rendement : 0,43 g ; 83 %.

Microanalyses :

| | Trouvé | Théorique |
|---|---|---|
| C | 24,26 | 24,9 |
| H | 2,68 | 2,85 |
| N | 7,13 | 7,74 |
| F | 11,37 | 11,80 |

**EXEMPLE 6 : RESULTATS**

Le temps de relaxation $T_1$ a été déterminé sur Minispec Brücker à 20 MHz, ou Jeol à 100 MHz à 37°C.

L'osmolarité a été mesurée par osmomètre (Wescor - Inc, 5100B) à 21°C et à concentration de 0,05 M.

On a effectué des études sur les tissus excisés de la manière suivante. Des organes sélectionnés tels que le foie, la rate, les reins, des échantillons de sang ont été recueillis sur des rats Wistar femelles qui ont été sacrifiés 15 minutes après injection intraveineuse de l'agent de contraste (IV) à une dose de 0,1 mmoles/kg.

La relaxivité du composé selon l'invention de formule IV (exemple 5) (ci-après Gd MNM-50) a été mesurée sur le minispec 20 MHz. Les résultats figurent au tableau 1 suivant.

Tableau 1

| Milieu | $T_1$ msec | $R_1$ $(mM^{-1}sec)^{-1}$ |
|---|---|---|
| Eau | 86* | 11,6 |
| Sérum albumine humaine (2,5 mg/100 ml) | 42 | 23,8 |
| Tampon acétate pH = 5,0.1 M | 90 | 11,1 |

* A 100 MHz, $T_1$ = 100msec, $R_1$ = 10 $(mM^{-1}sec)^{-1}$ en comparaison : avec le complexe $Gd(DTPA)^{2-}$ $T_1$ = 180 msec, $R_1$ = 5,5 $(mM^{-1}sec)^{-1}$ dans l'eau.

L'osmolarité du composé Gd(MNM-50) a été mesurée à 21°C à une concentration 0,05 M dans une solution physiologique (NaCl 0,9 %).

Gd(MNM-50) a une osmolarité de 353 mosmol/kg. Ce résultat est à comparer à l'osmolarité obtenue avec $Gd(DTPA)^{2-}$ dont l'osmolarité est égale à 398 mOsmol/kg, 0,2 ml Magnevist dilué à 2 ml avec la solution saline.

On note donc une relaxivité beaucoup plus importante avec le composé Gd(MNM-50) selon l'invention qu'avec le complexe $Gd(DTPA)^{2-}$, de même qu'une osmolarité nettement plus faible en faveur du Gd (MNM50).

Le tableau 2 ci-après donne les mesures in vitro de $T_1$ sur des tissus excisés de rats pour une dose injectée

de 0,1 mmol/kg de Gd (MNM-50) ou Gd(DTPA)$^{2-}$.

Tableau 2

| Composé Organes | Gd(MNM-50) msec | Gd(DTPA)$^{2-}$ msec | Contrôle msec |
|---|---|---|---|
| Foie | 139 | 597 | 742 |
| Sang | 138 | 782 | 1380 |
| Rate | 330 | 805 | 973 |
| Reins | 722 | 237 | 1230 |

La figure 1 représente une photographie de tissus de rats avant et après administration du composé Gd(MNM-50) à une dose de 0,1 mmol/kg. L'image après administration du Gd(MNM-50) (colonne de droite) a été prise 10 minutes après l'injection. Comme on peut le voir sur la figure, des contrastes importants sont obtenus entre le foie et les reins, d'une part, et les autres tissus, d'autre part. L'agent de contraste Gd (MNM50) se fixe d'ailleurs de façon tout à fait remarquable dans le foie.

## Revendications

**1)** Agent de contraste pour l'imagerie par RMN constitué d'un complexe entre un agent chélatant et un cation de métal paramagnétique, caractérisé en ce que ledit complexe répond à la formule I :

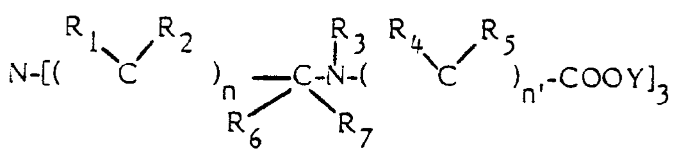

I

ou un sel physiologiquement acceptable de ce complexe, formule dans laquelle

– n, n' sont des nombres entiers identiques ou différents de 1 à 4

– les n et n' ($R_1$, $R_2$) et ($R_4$, $R_5$) respectivement identiques ou différents et $R_3$ et ($R_5$, $R_7$) sont choisis parmi H, un halogène, les groupes alkyl alcoxy, aryl, alkényl, cycloalkyl, arylalkyl éventuellement substitués par des groupes halogènes alcoxy, alkyl, nitril, nitro ou amino,

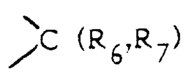

pouvant en outre représenter

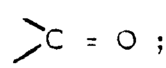

– les trois substituants Y sont identiques ou différents et représentent H ou un équivalent ionique de cation de métal paramagnétique divalent ou trivalent ou d'un cation physiologiquement acceptable provenant d'une base minérale ou organique,

avec la condition que deux au moins des substituants Y représentent des équivalents ioniques de cations de métaux paramagnétiques.

**2)** Agent de contraste selon la revendication 1 caractérisé en ce que

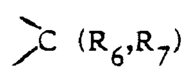

représente

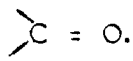

**3)** Agent de contraste selon l'une des revendications 1 ou 2, caractérisé en ce que le métal paramagnétique est choisi parmi les ions divalents ou trivalents des métaux de transition et des lanthanides.

**4)** Agent de contraste selon l'une des revendications 1 à 3, caractérisé en ce que le métal paramagnétique est choisi parmi $Co^{2+}$, $Mn^{2+}$, $Cu^{2+}$, $Cr^{3+}$, $Fe^{2+}$, $Fe^{3+}$, $Eu^{2+}$, $Gd^{3+}$, $Dy^{3+}$, $Ho^{3+}$.

**5)** Agent de contraste pour l'imagerie RMN selon l'une des revendications 1 à 4, constitué d'un complexe entre un agent chalétant et un cation de métal paramagnétique, caractérisé en ce qu'il répond à la formule II

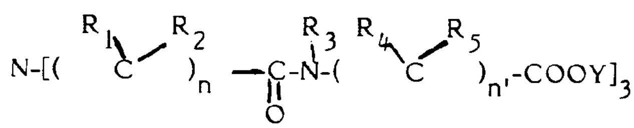

II

ou un sel d'acide physiologiquement acceptable de formule

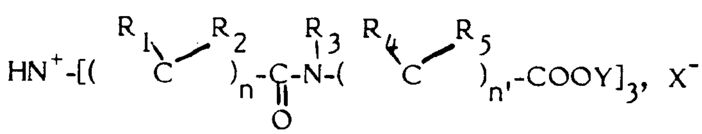

dans laquelle $X^-$ est l'anion provenant d'un acide de formule HX, formules dans lesquelles

– Y représente un équivalent ionique de métal paramagnétique trivalent,

– n, n', $R_1$, $R_2$, $R_3$ , $R_4$, $R_5$ ont les significations données précédemment.

**6)** Agent de contraste selon la revendication 5 caractérisé en ce que $X^-$ représente $Cl^-$ , $Br^-$, $F^-$, $I^-$, $C_nH_{2n+1}$ $COO^-$, $C_6H_5COO^-$, $C_nF_{2n+1}$ $COO^-$.

**7)** Agent de contraste selon l'une des revendications précédentes, caractérisé en ce que $R_3$ = H.

**8)** Agent de contraste selon l'une des revendications précédentes, caractérisé en ce que $R_1$ , $R_2$, $R_4$ et $R_5$ sont choisis parmi H, un halogène et un radical alkyle en $C_1$ à $C_4$ éventuellement substitué par un halogène.

**9)** Agent de contraste selon l'une des revendications précédentes, caractérisé en ce que $R_1 = R_2 = R_4 = R_5$ = H.

**10)** Agent de contraste selon l'une des revendications précédentes, caractérisé en ce que n = n' = 1.

**11)** Agent de contraste selon l'une des revendications 5 à 10, caractérisé en ce que $X^- = CF_3COO^-$.

**12)** Agent de contraste selon l'une des revendications 1 à 10, caractérisé en ce que le métal paramagnétique est l'ion Gadolinium $Gd^{3+}$.

**13)** Agent chélatant à structure peptidique pouvant former des complexes de métaux, caractérisé en ce qu'il répond à la formule

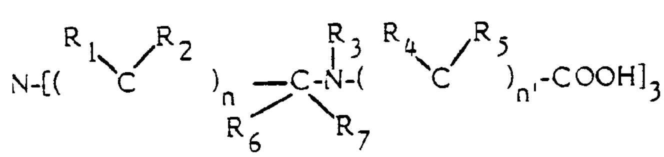

I

ou un sel physiologiquement acceptable de celui-ci, formule dans laquelle

– n, n' sont des nombres entiers identiques ou différents de 1 à 4

– les n et n' ($R_1$, $R_2$) et ($R_4$, $R_5$) respectivement identiques ou différents et $R_3$ et ($R_5$, $R_7$) sont choisis parmi H, un halogène, les groupes alkyl, alcoxy, aryl, alkényl, cycloalkyl éventuellement substitués par des groupes halogènes alcoxy, alkyl, nitril, nitro ou amino,

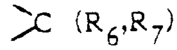

pouvant en outre représenter

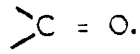

**14)** Agent chélatant selon la revendication 13, caractérisé en ce qu'il répond à la formule :

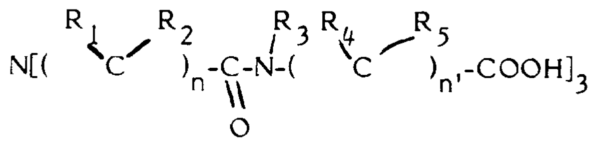

ou un sel physiologiquement acceptable de celui-ci, formule dans laquelle n, n', $R_1$, $R_2$, $R_3$, $R_4$, $R_5$ ont les significations données dans l'une des revendications 1 à 11.

**15)** Agent chélatant selon la revendication 14, caractérisé en ce qu'il a pour formule

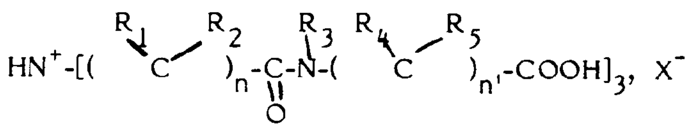

ou $X^-$ provient d'un acide HX tel que défini dans les revendications 2 à 11.

**16)** Complexe métallique formé par un agent chélateur selon l'une des revendications 14 et 15 et un ou des cation(s) métallique(s).

**17)** Procédé de préparation d'un agent de contraste pour RMN selon l'une des revendications 1 à 12, caractérisé en ce que l'on mélange en solution aqueuse un agent chélateur ou un de ses sels selon l'une des revendications 13 à 15, avec un composé dudit métal paramagnétique modérément soluble ou en suspension en phase aqueuse.

**18)** Procédé de préparation d'un agent chélatant selon l'une des revendications 13 à 15, caractérisé en ce que l'on fait réagir un composé de formule

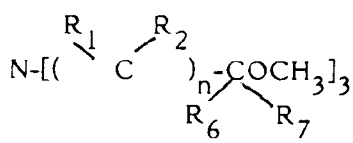

avec un composé de formule :

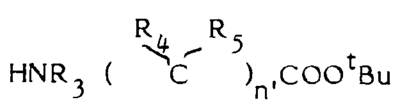

pour obtenir le composé de formule :

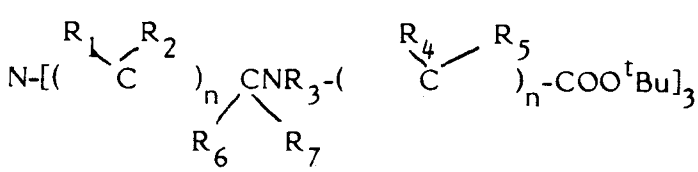

puis l'on déprotège les fonctions acides éventuellement en faisant réagir le composé avec un acide pour obtenir un sel d'ammonium.

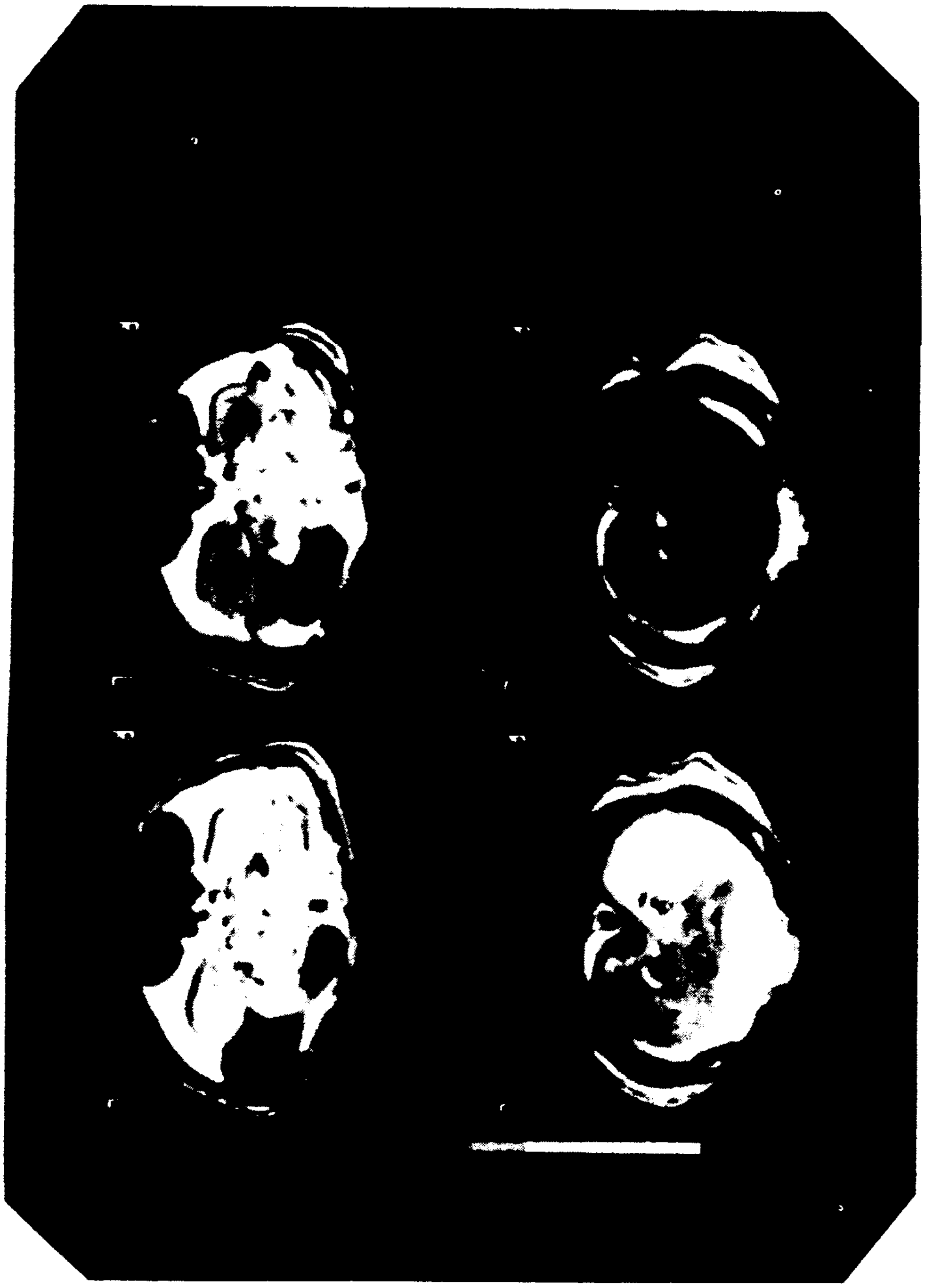

REIN

FOIE

Office européen des brevets

# RAPPORT DE RECHERCHE EUROPEENNE

Numero de la demande

EP 91 40 1697

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EP-A-0 326 226 (NYCOMED)<br>* Exemples; revendications *<br>--- | 1-18 | C 07 K 5/06<br>A 61 K 49/00 |
| A | WO-A-9 001 024 (MALLINCKRODT)<br>* Exemples; revendications *<br>--- | 1-18 | |
| A | WO-A-9 003 804 (SALUTAR)<br>* Exemples; revendications *<br>--- | 1-18 | |
| A | WO-A-8 602 352 (BOARD OF REGENTS, THE UNIVERSITY OF TEXAS SYSTEM)<br>* En entier *<br>--- | 1-18 | |
| D,A | US-A-4 647 447 (GRIES et al.)<br>* En entier *<br>----- | 1-18 | |
| | | | **DOMAINES TECHNIQUES RECHERCHES (Int. Cl.5)** |
| | | | C 07 K 5/00 |

Le présent rapport a été établi pour toutes les revendications

| Lieu de la recherche | Date d'achèvement de la recherche | Examinateur |
|---|---|---|
| LA HAYE | 25-09-1991 | HELPS I.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)